# EUROPÄISCHE PATENTSCHRIFT

(11) **EP 2 229 887 B1**
(45) Veröffentlichungstag und Bekanntmachung des Hinweises auf die Patenterteilung: **20.07.2011**
(21) Anmeldenummer: 10168148.4
(22) Anmeldetag: 20.05.2005
(51) Int. Cl.: A61B 5/15

(54) **Lanzettensystem mit Sterilschutz**
Lancet device with sterile protection
Dispositif de lancettes avec protection stérile

(43) Veröffentlichungstag der Anmeldung: 22.09.2010
(62) Teilanmeldung aus: 05011038.6
(73) Patentinhaber: F. Hoffmann-La Roche AG, 4070 Basel (CH); Roche Diagnostics GmbH, 68305 Mannheim (DE)
(72) Erfinder: List, Hans, 64754 Hesseneck-Kailbach (DE)
(74) Vertreter: Stößel, Matthias

(56) Entgegenhaltungen:
- WO-A-95/10977
- US-A- 5 318 581
- US-A- 5 636 640
- US-B1- 6 540 762

## Beschreibung

Die vorliegende Erfindung betrifft ein Lanzettensystem mit einer Lanzettenspitze, einem Lanzettenkörper und einem Körper, der als Sterilschutz für die Lanzettenspitze dient. Des Weiteren betrifft die Erfindung eine Stechhilfe mit mindestens einem erfindungsgemäßen Lanzettensystem und ein als Magazin mit einer Vielzahl von Lanzetten vorgesehenes Lanzettensystem.

Eine Entnahme von Körperflüssigkeiten, insbesondere von Blut, wird vor allem mit dem Ziel einer nachfolgenden Analyse durchgeführt, um eine Diagnose von Krankheiten oder die Überwachung des Stoffwechselzustandes eines Patienten zu ermöglichen. Insbesondere von Diabetikern wird eine solche Entnahme durchgeführt, um die Blutzuckerkonzentration zu bestimmen. Um für Diagnosezwecke eine Entnahme von nur geringen Mengen Blut vorzunehmen, werden üblicherweise sterile, scharfe Lanzetten verwendet, die z. B. von Krankenhauspersonal oder dem Patienten selbst bei dem Patienten in die Fingerbeere oder auch andere Körperteile kurz eingestochen werden. Vor allem im Bereich des so genannten "Home-Monitoring", wobei medizinische Laien selbst einfache Analysen des Blutes durchführen, werden Lanzetten und dazu passende Geräte (so genannte Blutentnahmegeräte, Blutlanzettenvorrichtungen oder - wie sie im Folgenden genannt werden sollen - Stechhilfen), angeboten, die eine möglichst schmerzarme und reproduzierbare Blutgewinnung ermöglichen.

Die Lanzettenspitze von zur Blutgewinnung verwendeten Lanzetten wird typischerweise im voraus sterilisiert und durch einen Sterilschutz (z. B. in Form einer Kappe oder Tasche) in einem sterilen Zustand gehalten, bevor sie für einen Stechvorgang verwendet wird, um sicherzustellen, dass die Lanzettenspitze durch die Umgebung nicht kontaminiert wird. Ferner werden häufig Vorkehrungen dafür getroffen, dass die Lanzettenspitze nach einem erfolgten Stechvorgang abgeschirmt wird (gegebenenfalls durch dieselbe Kappe oder Tasche), so dass unbeabsichtigte Verletzungen und damit einhergehende Infektionen durch an der Lanzettenspitze anhaftendes Blut vermieden werden.

Bei Einzellanzetten kann beispielsweise ein Sterilschutz hergestellt werden, indem die Spitze der Lanzette in einem Arbeitsgang mit der Erzeugung des Lanzettenkörpers mit Kunststoff umspritzt wird. Vor Gebrauch entfernt der Anwender dieses Teil manuell, meist beim Einsetzen in eine Stechhilfe. Im Fall von magazinierten Lanzetten sind ähnliche Sterilschutzeinrichtungen gebräuchlich, z. B. solche, bei denen die Lanzette nach hinten aus einem Sterilschutz herausgezogen wird, woraufhin der Sterilschutz per Federkraft aus dem Stichweg befördert wird. Hierfür sind relativ aufwändige Mechaniken erforderlich, insbesondere Federn, die in das Verbrauchsmaterial integriert sind.

In WO 01/66010 wird die Umständlichkeit dieser Mechanik umgangen, indem der Sterilschutz einfach durchstochen wird. WO 01/66010 bezieht sich auf eine Lanzette enthaltend eine Lanzettennadel mit einer Spitze und einen Lanzettenkörper, der die Lanzettennadel zumindest im Bereich der Spitze vollständig umgibt. Der Lanzettenkörper besteht zumindest im Bereich der Spitze der Lanzettennadel aus einem elastischen Material, in das die Spitze der Lanzettennadel eingebettet ist. Ferner wird eine Lanzette beschrieben, die eine Lanzettennadel mit einer Spitze und einen Hohlkörper, der zumindest die Spitze der Lanzettennadel umgibt, enthält. Die Lanzettennadel ist im Bereich ihrer Spitze im Hohlkörper beweglich und der Hohlkörper besteht zumindest teilweise aus einem elastischen Material, das von der Spitze der Lanzettennadel beim Stechvorgang durchstoßen werden kann und das sich gegebenenfalls nach dem Zurückziehen der Spitze der Lanzettennadel in den Hohlkörper wieder verschließt.

DE 28 03 345 betrifft ein Blutprobenentnahmegerät mit einer unter Krafteinwirkung gegen die Körperoberfläche eines Patienten bewegbaren Nadel sowie mit einer zur Krafteinwirkung auf die Nadel in Richtung der Nadelspitze eingerichteten Betätigungseinrichtung mit Stößel und Auslöser. Als Nadeln werden Blutlanzetten verwendet, wobei diese einzeln in Taschen einer Streifenpackung aufgenommen sind und mittels einer an der Streifenpackung angreifenden Transporteinrichtung in das Blutprobenentnahmegerät eingeführt sowie nach Gebrauch wieder entnommen werden und wobei der Stößel auf die in den Taschen aufgenommenen Blutlanzetten einwirkt. In diesem Blutprobenentnahmegerät ist entweder eine Schneideeinrichtung enthalten, durch welche vor der Krafteinwirkung der spitzenseitige Randbereich der Streifenpackung abtrennbar ist, oder die Blutlanzetten perforieren die Streifenpackung unter der Krafteinwirkung selbst.

In US 2003/0199893 A1 ist ebenfalls eine Schneidvorrichtung vorgesehen, um mit erheblichem mechanischen Aufwand eine ein Eindringelement umgebende Sterilitätsbarriere vor einem Stechvorgang aufzuschneiden, oder das Eindringelement selbst durchdringt die Sterilitätsbarriere vor einem Stechvorgang.

Patentschrift WO 95/010977 A beschreibt ein Lanzettensystem lauf Oberbegriff des Anspruchs 1.

In der Regel sollten Lanzettensysteme, nachdem mit ihnen einmal ein Stechvorgang ausgeübt worden ist, nicht wieder verwendet werden. Im Home-Monitoring ist es jedoch denkbar, dass ein einmal in eine Stechhilfe eingelegtes Lanzettensystem durch denselben Benutzer mehrfach verwendet wird, bevor es verworfen und durch ein neues Lanzettensystem ersetzt wird. Im Falle eines beim ersten Stechvorgangs zum Öffnen durch die Lanzette selbst durchstochenen Sterilschutzes gelangt der Sterilschutz nach dem Stechvorgang zumindest teilweise wieder vor die in den Sterilschutz zurückbewegte Lanzettenspitze. Bei einer erneuten Benutzung dieser bereits benutzten Lanzette besteht die Gefahr, dass die ursprünglich geschaffene Öffnung im Sterilschutz nicht exakt wieder getroffen wird, wodurch ein erneuter Durchstoß erfolgt. Dafür ist die Lanzettenspitze nicht ausgelegt, sondern für einen schmerzarmen Einstich in die Haut. Bei jedem Durchstoßen von z. B. Kunststoffmaterialien leidet die Schärfe der Lanzette ein wenig. Zum anderen werden nach mehreren solchen Vorgängen evt. Teile des Sterilschutzes abgetrennt und gelangen möglicherweise mit der Lanzettenspitze in die Einstichwunde, was sicher vermieden werden sollte.

Des Weiteren haben die im Stand der Technik bekannten Lösungen den Nachteil, dass (auch bei nur einmaliger Verwendung einer Lanzette) beim Zurückziehen der Lanzette durch ihre Austrittsöffnung in ihren ursprünglichen Sterilschutz nach dem Stechvorgang an der Lanzettenspitze haftende Blutreste an dem Rand der Austrittsöffnung in dem Sterilschutz abgestreift werden und so an der Außenseite des Sterilschutzes haften bleiben, wodurch eine unerwünschte Kontamination der Umgebung verursacht wird.

Die Aufgabe der vorliegenden Erfindung ist es, die Nachteile des Standes der Technik zu beseitigen. Insbesondere ist es die Aufgabe der vorliegenden Erfindung, Lanzettensysteme oder Lanzettenmagazine bereitzustellen, bei denen zumindest die Lanzettenspitze im unbenutzten Zustand bis unmittelbar vor der ersten Benutzung steril gehalten wird und die Lanzette nach dieser Benutzung so aufbewahrt wird, dass eine Kontamination der Umgebung und eine unbeabsichtigte Verletzung des Benutzers vermieden wird.

Diese Aufgabe wird erfindungsgemäß gelöst durch ein Lanzettensystem enthaltend eine Lanzettenspitze, einen Lanzettenkörper und einen Körper, der zumindest die Lanzettenspitze umgibt, wobei die Lanzettenspitze in dem Körper beweglich ist, wobei der Körper ein plastisches Material enthält, das von der Lanzettenspitze bei einem Stechvorgang zur Herstellung einer Austrittsöffnung durchstoßbar ist, und wobei der Lanzettenkörper eine Verdickung aufweist, die so ausgebildet ist, dass sie die Austrittsöffnung bei dem Stechvorgang aufweitet, und wobei die Lanzettenspitze nach dem Stechvorgang in den Körper zurückbewegbar ist.

Das erfindungsgemäße Lanzettensystem weist mindestens eine Lanzette mit einer Lanzettenspitze und einem Lanzettenkörper auf. Gemäß einer bevorzugten Ausführungsform der vorliegenden Erfindung umfasst das Lanzettensystem eine Vielzahl von Lanzetten. Dabei kann das Lanzettensystem als Magazin für Lanzetten in eine Stechhilfe eingesetzt werden.

Die Lanzettenspitze wird beim bestimmungsgemäßen Gebrauch des Lanzettensystems in Gewebe eingestochen, um den Ausfluss einer Körperflüssigkeit, insbesondere von Blut oder interstitieller Flüssigkeit, zu verursachen. Die Lanzettenspitze kann z. B. rotationssymmetrisch ausgebildet sein. Es können auch ein oder mehrere Schliffe an der Lanzettenspitze angebracht sein. Die hierfür vorhandenen, zur Längsachse der Lanzettenspitze geneigten und zu der Spitze zulaufenden Kanten dienen beim Einstich als scharfe Schneide und gestalten den Stechvorgang in vorteilhafter Weise schmerzärmer, als dies mit rotationssymmetrischen Lanzettenspitzen der Fall ist.

Der Körper, der zumindest die Lanzettenspitze umgibt, dient als Sterilschutz für die Lanzettenspitze. Die Lanzettenspitze ist dadurch im unbenutzten Zustand keimdicht abgeschirmt, so das Keime bis unmittelbar vor der (ersten) Benutzung des erfindungsgemäßen Lanzettensystems nicht an die Lanzettenspitze gelangen können. Nach einer geeigneten Sterilisierung bleibt die Lanzettenspitze über lange Zeit steril.

Der Körper enthält ein plastisches Material, das von der Lanzettenspitze bei einem Stechvorgang zur Herstellung einer Austrittsöffnung durchstoßbar ist. Plastisch bedeutet in diesem Zusammenhang, dass das Material die Form weitgehend beibehält, die ihm durch eine äußere Krafteinwirkung gegeben wurde. Damit verschließt sich z. B. die Austrittsöffnung in dem plastischen Material, die die Lanzettenspitze beim Durchstoßen erzeugt, nach einem Zurückziehen der Lanzettenspitze aus der Austrittsöffnung nicht wieder, wie dies bei einem elastischen Material der Fall wäre. Bei dem plastischen Material ist die Streckgrenze niedrig relativ zur Bruchgrenze des Materials.

Das plastische Material kann z. B. als Folie, bestehend aus einem Werkstoff, vorlieben oder schichtweise aus verschiedenen plastischen Werkstoffen aufgebaut sein. Bei einem Stechvorgang durchstößt die Lanzettenspitze des erfindungsgemäßen Lanzettensystems das plastische Material und tritt durch die Austrittsöffnung aus dem Körper aus, um einen Einstich im Gewebe zur Entnahme einer Körperflüssigkeit durchzuführen.

Der Lanzettenkörper weitet dabei die Austrittsöffnung durch seine Verdickung auf, die ebenfalls durch die Austrittsöffnung hindurchgestoßen wird. Eine Verdickung ist in diesem Zusammenhang ein Bereich des Lanzettenkörpers, der einen gegenüber der Lanzettenspitze größeren Durchmesser aufweist und der durch die Austrittsöffnung bewegbar ist. Dies hat den Vorteil, dass nach dem Einstechvorgang, wenn die Lanzettenspitze wieder in den Körper zurückgezogen wird, in dem plastischen Material des Körpers einer Austrittsöffnung erhalten bleibt, deren Durchmesser größer als der der Lanzettenspitze ist. Daher streift die Lanzettenspitze beim Zurückbewegen in den Körper nicht an den Rändern der Austrittsöffnung entlang und kontaminiert diesen außen an dem Lanzettensystem liegenden Bereich nicht mit an der Lanzettenspitze verbleibenden Resten der Körperflüssigkeit. Ferner stößt die Lanzettenspitze beim erneuten Austreten aus der Austrittsöffnung nicht ein weiteres Mal auf das plastische Material und wird daher nicht durch ein weiteres Durchstechen von Material beansprucht oder durch gegebenenfalls abgetrennte Teile des Materials oder an dem Material haftende Keime verschmutzt. Die Gefahr einer Kontamination der bei dem Stechvorgang erzeugten Wunde durch eine kontaminierte Lanzettenspitze wird somit verringert. Nach jedem Stechvorgang wird die Lanzettenspitze in den Körper zurückbewegt. Der Benutzer des erfindungsgemäßen Lanzettensystems ist durch diese Maßnahme vor einer unbeabsichtigten Verletzung an der benutzten Lanzettenspitze geschützt.

Bei dem Stechvorgang kann der Lanzettenkörper entweder gekoppelt an die Lanzettenspitze zur Aufweitung nach dieser durch die Austrittsöffnung bewegt werden oder die Verdickung an dem Lanzettenkörper kann unabhängig von der Lanzettenspitze zur Aufweitung nach der Spitze durch die Austrittsöffnung bewegt werden. Im ersten Fall besteht vorzugsweise eine feste räumliche Anordnung zwischen Lanzettenspitze und Lanzettenkörper und im zweiten Fall ist der Lanzettenkörper vorzugsweise relativ zu der Lanzettenspitze beweglich.

Gemäß einer bevorzugten Ausführungsform der vorliegenden Erfindung ist die Verdickung ein konischer Bereich des Lanzettenkörpers, dessen Radius mit zunehmendem Abstand von der Lanzettenspitze zunimmt. Ein konischer Bereich hat den Vorteil, dass eine Aufweitung der Austrittsöffnung beim Hindurchschieben durch die Austrittsöffnung sukzessive durch Aufbringen einer geringeren Kraft erreicht werden kann, als z. B. durch eine wulstartige Verdickung.

Vorzugsweise ist die Verdickung bei dem erfindungsgemäßen Lanzettensystem in einem an die Lanzettenspitze angrenzenden Bereich angeordnet. Dadurch kann die Verdickung direkt nach dem Durchstoßen des plastischen Materials mit der Lanzettenspitze ohne räumlichen oder zeitlichen Abstand die Aufweitung der Austrittsöffnung bewirken.

Das plastische Material weist vorzugsweise eine hohe Reißdehnung, eine niedrige Festigkeit (Übergang vom elastischen zum plastischen Verhalten), ein hohes E-Modul und eine niedrige Kugeldruckhärte auf. Die Reißdehnung des plastischen Materials liegt bevorzugt über 50%. Die Kugeldruckhärte des plastischen Materials liegt bevorzugt unter 50 N/mm². Insbesondere sollte das plastische Material weich relativ zu dem Material der Lanzettenspitze sein, damit diese nicht beim Durchstoßen des plastischen Materials beschädigt wird. Für eine Lanzettenspitze z.B. aus Stahl mit einer Vickers-Härte von 250 sollte die Vickers-Härte des plastischen Materials bevorzugt unter 25 liegen. Gemäß einer bevorzugten Ausführungsform der vorliegenden Erfindung enthält das plastische Material mindestens ein Material ausgewählt aus der Gruppe LD(low density)-Polyethylen -, HMW(high molecular weigth)-Polyethylen oder Polypropylen, insbesondere wenn der Körper einstückig mit dem plastischen Material gefertigt wird (z.B. durch Spritzgießen). Vorzugsweise enthält das plastische Material mindestens ein Material ausgewählt aus der Gruppe LD(low density)-Polyethylen, HMW(high molecular weigth)-Polyethylen, Polypropylen, Aluminium, Zink oder Polytetrafluorethylen, insbesondere wenn der Körper als rohrförmiger Hohlkörper ausgebildet ist und das plastische Material in Form einer eine Öffnung des Körpers verschließenden Folie vorliegt. Falls die Folie aus einem Metall gefertigt ist, weist sie vorzugsweise zusätzlich einen Haftkleber (ggf. in Form einer Beschichtung der Folie z.B. mit heiß klebbarem Polyethylen) auf, mit dessen Hilfe sie an dem Körper befestigt werden kann.

In einer bevorzugten Ausführungsform der Erfindung enthält das Lanzettensystem eine Lanzettennadel, die die Lanzettenspitze und einen Nadelkörper umfasst, wobei der

Lanzettenkörper den Nadelkörper zumindest teilweise umgibt. Der Lanzettenkörper kann dabei fest mit dem Nadelkörper verbunden sein oder der Nadelkörper kann (zur Durchführung des Stechvorganges) relativ zu dem Lanzettenkörper beweglich sein. Der Lanzettenkörper kann den Nadelkörper teilweise oder vollständig umgeben. Die Lanzettennadel des erfindungsgemäßen Lanzettensystems ist aus einem Material gefertigt, das ausreichend hart ist, um eine mechanische Beanspruchung während des Stechvorgangs, insbesondere während des Durchstoßens des plastischen Materials und während der Bearbeitungsschritte oder evt. sonstige Beanspruchungen ohne Deformation zu überstehen. Weiterhin muss das Material so beschaffen sein, dass von der Lanzettennadel während des Stechvorgangs keine Partikel abbrechen oder sich ablösen. Schließlich muss das Lanzettennadelmaterial auch so bearbeitbar sein, dass die Lanzettenspitze ausreichend spitz und die Kanten der Lanzettenspitze gegebenenfalls ausreichend scharf geschliffen werden können. Gut geeignete Materialien für die Lanzettennadel sind vor allem Metalle und von diesen insbesondere Edelstähle. Es sind jedoch auch Nadeln aus Keramik oder Kunststoffen möglich.

Der Lanzettenkörper ist relativ zu dem Körper beweglich, damit seine Verdickung zur Aufweitung der durch die Lanzettenspitze erzeugten Austrittsöffnung zumindest teilweise ebenfalls durch diese hindurchbewegt werden kann.

In einer bevorzugten Ausführungsform umgibt der Körper den Lanzettenkörper und die Lanzettenspitze vollständig, wobei der Lanzettenkörper und die Lanzettenspitze in dem Körper gemeinsam verschiebbar sind. Dabei sind der Lanzettenkörper und die Lanzettenspitze bevorzugt in Längsrichtung in Bezug auf die Lanzettenspitze verschiebbar.

Für das getrennte oder gemeinsame Bewegen von Lanzettenspitze und Lanzettenkörper können in einer Stechhilfe, in der das erfindungsgemäße Lanzettensystem zum Einsatz kommt, die geeigneten konstruktiven Maßnahmen (z. B. Betätigungsmittel, Antriebs- oder Halterungselemente) vorgesehen werden.

Gemäß einer bevorzugten Ausführungsform der vorliegenden Erfindung ist der Körper als rohrförmiger Hohlkörper ausgebildet, mit einem ersten durch eine erste Folie verschlossenen Ende und mit einem zweiten durch eine zweite Folie verschlossenen Ende, wobei mindestens eine der zwei Folien aus dem plastischen Material besteht. Beim (ersten) Stechvorgang mit der Lanzette durchstößt die Lanzettenspitze eine der Folien (die aus dem plastischen Material besteht), um eine Austrittsöffnung zu erzeugen, durch die die Spitze aus dem Körper austreten kann. Vorzugsweise kann die erste Folie bei dieser Ausführungsform durch ein Betätigungsmittel geöffnet werden, das zur Bewegung der Lanzettenspitze zu der zweiten Folie hin dient, wobei die zweite Folie aus dem plastischen Material besteht und durch die Lanzettenspitze durchstoßbar ist.

Des Weiteren kann der Körper zumindest teilweise ein Kunststoffkörper sein, der durch ein Umspritzen zumindest der Lanzettenspitze mit dem Kunststoff in einem Spritzgussverfahren hergestellt wird. Der Körper kann weiterhin vollständig aus dem plastischen Material bestehen. Eine weitere Möglichkeit ist, dass der Körper nur in dem Bereich, in dem er beim Stechvorgang von der Lanzettenspitze durchstoßen wird, aus dem plastischen Material besteht und in den Bereichen, die nicht mit der Lanzettenspitze in Berührung kommen, aus einem anderen, vorzugsweise einem steifen, spritzgussfähigen Material gefertigt ist.

Die Erfindung bezieht sich weiterhin auf ein als Magazin gestaltetes Lanzettensystem mit mindestens zwei Lanzetten, die jeweils eine Lanzettenspitze und einen Lanzettenkörper aufweisen und die jeweils in einzelnen, voneinander unabhängigen Kammern des Lanzettensystems enthalten sind, wobei jede Kammer zumindest eine Kammeröffnung aufweist, wobei die Kammeröffnung durch ein plastisches Material verschlossen ist, das von der Lanzettenspitze bei einem Stechvorgang zur Herstellung einer Austrittsöffnung durchstoßbar ist, und wobei der Lanzettenkörper eine Verdickung aufweist, die so ausgebildet ist, dass sie die Austrittsöffnung bei dem Stechvorgang aufweitet und wobei die Lanzettenspitze nach dem Stechvorgang in die Kammer zurückbewegbar ist.

Das als Magazin vorgesehene Lanzettensystem dient zur Aufbewahrung unbenutzter und benutzter Lanzetten. Die Kammern des Magazins übernehmen die Funktion des Körpers des oben beschriebenen erfindungsgemäßen Lanzettensystems. Die Kammern sind vorzugsweise in dem Magazin regelmäßig geometrisch angeordnet, wobei benachbarte Kammern gemeinsame Wände aufweisen können. Das Magazin kann z. B. stapel-, scheiben- oder trommelförmig aufgebaut sein. Bis unmittelbar vor der (ersten) Benutzung werden die Lanzettenspitzen in ihrer jeweiligen Kammer steril aufbewahrt. Bei einem Stechvorgang durchstößt die Lanzettenspitze eines Lanzettensystems das plastische Material, das die Kammeröffnung ihrer Kammer keimdicht verschließt, und die Verdickung des Lanzettenkörpers weitet die dabei entstehende Austrittsöffnung auf. Nach dem Stechvorgang wird die Lanzettenspitze wieder durch die Austrittsöffnung in ihre Kammer zurückbewegt, so dass ein unbeabsichtigtes Verletzen an der Lanzettenspitze ausgeschlossen wird. Die sich durch das erfindungsgemäße Lanzettensystem mit einer Vielzahl von Lanzetten ergebenden Vorteile entsprechen unter anderem den bereits für das erfindungsgemäße Lanzettensystem mit einer Lanzette genannten.

Die Erfindung bezieht sich weiterhin auf eine Stechhilfe, enthaltend mindestens ein erfindungsgemäßes Lanzettensystem und ein Betätigungsmittel, das auf das Lanzettensystem zur Bewegung der Lanzettenspitze in dem Körper oder der Kammer einwirken kann, so dass die Lanzettenspitze zur Durchführung eines Stechvorgangs das plastische Material durchstößt und aus der Austrittsöffnung austritt. In die Stechhilfe werden die erfindungsgemäßen Lanzettensysteme einzeln manuell durch den Benutzer eingelegt. Es kann aber auch eine Vielzahl von Lanzetten (aufbewahrt in einem erfindungsgemäßen, als Magazin dienenden Lanzettensystem) in der Stechhilfe bereitgestellt werden. Ein in der Stechhilfe enthaltenes Betätigungsmittel (z. B. eine Art Stößel oder Haken) wirkt in der Stechhilfe auf das Lanzettensystem, um die Lanzettenspitze zum Durchstoßen des plastischen Materials und zur Ausführung des Stechvorganges zu bewegen. Das Betätigungsmittel kann ferner dazu dienen, die Lanzettenspitze nach einem Stechvorgang in ihren Körper bzw. in ihre Kammer zurückzubewegen. Es kann zu diesem Zweck jedoch auch ein weiteres Element (z. B. eine Feder) in der Stechhilfe vorgesehen sein. Des Weiteren kann das Betätigungsmittel ebenfalls eine Bewegung der Verdickung des Lanzettenkörpers durch die Austrittsöffnung bewirken, um die Aufweitung der Austrittsöffnung zu erzielen.

Die Erfindung wird anhand der Zeichnung nachstehend näher erläutert.

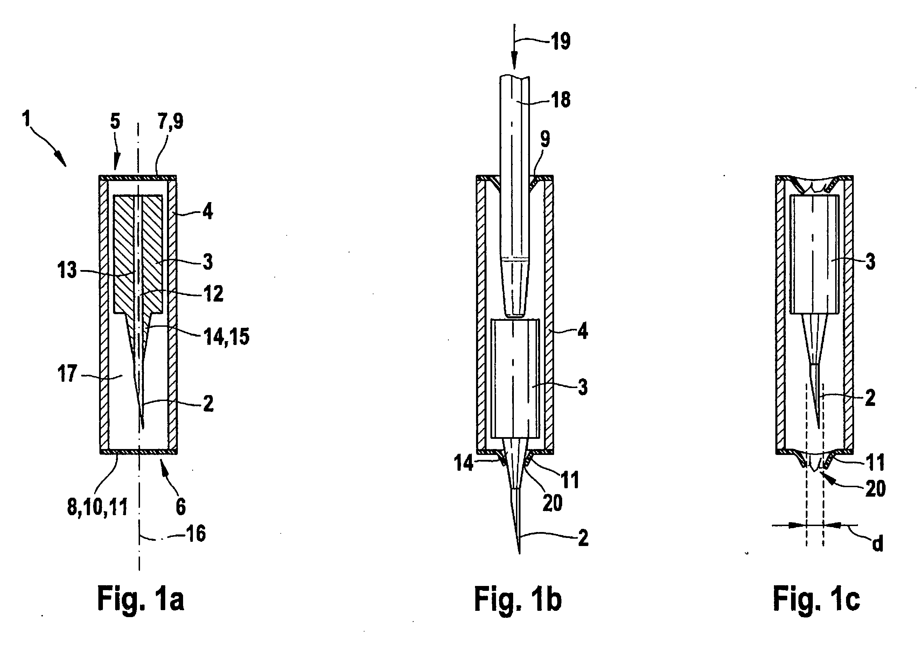

Figur 1 zeigt schematisch ein erfindungsgemäßes Lanzettensystem und den Ablauf eines Stechvorganges mit diesem Lanzettensystem.

Das Lanzettensystem 1 aus Figur 1 weist eine Lanzettenspitze 2, einen Lanzettenkörper 3 und einen Körper 4 auf. In Figur 1a) ist das Lanzettensystem 1 vor dem (ersten) Gebrauch im Schnitt dargestellt (Symmetrielinie 16). Der Körper 4 ist als rohrförmiger Hohlkörper ausgebildet, wobei (vor dem ersten Stechvorgang) beide Enden 5, 6 des Körpers 4 keimdicht durch zwei Verschlüsse 7, 8 verschlossen sind. Die beiden Verschlüsse 7, 8 können beispielsweise zwei Folien 9, 10 sein. Zumindest der zweite Verschluss 8 besteht aus einem plastischen Material 11.

Das Lanzettensystem 1 enthält eine Lanzettennadel 12, die die Lanzettenspitze 2 und einen Nadelkörper 13 umfasst. Der Lanzettenkörper 3 umgibt den Nadelkörper 13 und weist eine Verdickung 14 in Form eines konischen Bereichs 15 auf, dessen Radius mit zunehmendem Abstand von der Lanzenspitze 2 zunimmt. Die Verdickung 14 ist in einem an die Lanzettenspitze 2 angrenzenden Bereich angeordnet.

Der Körper 4 umgibt den Lanzettenkörper 3 und die Lanzettenspitze 2 vollständig, und der Lanzettenkörper 3 und die Lanzettennadel 12 (inklusive Lanzettenspitze 2) sind fest miteinander verbunden. Sie sind gemeinsam in dem Hohlraum 17 des Körpers 4 verschiebbar.
Figur 1b) zeigt den (ersten) Stechvorgang, der mit dem dargestellten erfindungsgemäßen Lanzettensystem ausgeführt wird. Ein Betätigungsmittel 18 übt eine Kraft in Stechrichtung 19 auf den Lanzettenkörper 3 und die Lanzettenspitze 2 aus. Dazu durchstößt das Betätigungsmittel 18 die erste Folie 9, um in den Hohlraum 17 des Körpers 4 zu gelangen. Dann schiebt das Betätigungsmittel 18 die Lanzettenspitze 2 in Richtung des plastischen Materials 11, so dass das plastische Material 11 von der Lanzettenspitze 2 durchstoßen und eine Austrittsöffnung 20 erzeugt wird. Während sich die Lanzettenspitze 2 weiter in Stechrichtung 19 bewegt, wird die Verdickung 14 in Stechrichtung 19 durch die Austrittsöffnung 20 geschoben und weitet diese aufgrund ihres zunehmenden Querschnitts auf.
Figur 1c) zeigt die in dem Hohlraum 17 des Körpers 4 nach dem Stechvorgang zurückgezogene Lanzettenspitze 2. Das Zurückziehen kann z. B. durch das Betätigungsmittel 18 bewirkt werden, das sich an den Lanzettenkörper 3 anheftet (z. B. mit diesem verhakt) und entgegen der Stechrichtung 19 zieht. Das plastische Material 11 weist nun eine aufgeweitete Austrittsöffnung 20 auf, die aufgrund der Eigenschaften des plastischen Materials in diesem aufgeweiteten Zustand bleibt. Der Durchmesser d der Austrittsöffnung ist dabei so groß, dass die Lanzettenspitze 2 das plastische Material 11 weder beim Zurückbewegen in den Körper 4 noch bei einem möglicherweise weiteren Stechvorgang berührt, so dass eine Kontamination oder Beanspruchung der Lanzettenspitze 2 durch das plastische Material 11 ebenso vermieden wird wie eine Kontamination der Umgebung durch ein Abstreifen von Körperflüssigkeitsresten an dem plastischen Material 11, die an der Lanzettenspitze 2 nach jedem Stechvorgang haften.

### Bezugszeichenliste

- 1: Lanzettensystem
- 2: Lanzettenspitze
- 3: Lanzettenkörper
- 4: Körper
- 5: erstes Ende des Körpers
- 6: zweites Ende des Körpers
- 7: erster Verschluss
- 8: zweiter Verschluss
- 9: erste Folie
- 10: zweite Folie
- 11: plastisches Material
- 12: Lanzettennadel
- 13: Nadelkörper
- 14: Verdickung
- 15: konischer Bereich
- 16: Symmetrielinie
- 17: Hohlraum
- 18: Betätigungsmittel
- 19: Stechrichtung
- 20: Austrittsöffnung

## Patentansprüche

1. Lanzettensystem (1), enthaltend eine Lanzettenspitze (2), einen Lanzettenkörper (3) und einen Körper (4), der zumindest die Lanzettenspitze (2) umgibt, wobei die Lanzettenspitze (2) in dem Körper (4) beweglich ist, wobei der Körper (4) ein plastisches Material (11) enthält, das von der Lanzettenspitze (2) bei einem Stechvorgang zur Herstellung einer Austrittsöffnung (20) durchstoßbar ist, **dadurch gekennzeichnet, dass** der Lanzettenkörper (3) eine Verdickung (14) aufweist, die so ausgebildet ist, dass sie die Austrittsöffnung (20) bei dem Stechvorgang aufweitet, und dass die Lanzettenspitze (2) nach dem Stechvorgang in den Körper (4) zurückbewegbar ist.

2. Lanzettensystem gemäß Anspruch 1, **dadurch gekennzeichnet, dass** die Verdickung (14) ein konischer Bereich (15) des Lanzettenkörpers (3) ist, dessen Radius mit zunehmendem Abstand von der Lanzettenspitze (2) zunimmt.

3. Lanzettensystem gemäß einem der Ansprüche 1 oder 2, **dadurch gekennzeichnet, dass** die Verdickung (14) in einem an die Lanzettenspitze (2) angrenzenden Bereich angeordnet ist.

4. Lanzettensystem gemäß einem der Ansprüche 1 bis 3, **dadurch gekennzeichnet, dass** das plastische Material (11) mindestens ein Material ausgewählt aus der Gruppe Polyethylen, Polypropylen, Aluminium, Zinn und Polytetrafluorethylen enthält.

5. Lanzettensystem gemäß einem der Ansprüche 1 bis 4, **dadurch gekennzeichnet, dass** das Lanzettensystem (1) eine Lanzettennadel (12) enthält, die die Lanzettenspitze (2) und einen Nadelkörper (13) umfasst, wobei der Lanzettenkörper (3) den Nadelkörper (13) zumindest teilweise umgibt.

6. Lanzettensystem gemäß einem der Ansprüche 1 bis 5, **dadurch gekennzeichnet, dass** der Körper (4) den Lanzettenkörper (3) und die Lanzettenspitze (2) vollständig umgibt, wobei der Lanzettenkörper (3) und die Lanzettenspitze (2) in dem Körper (4) gemeinsam verschiebbar sind.

7. Lanzettensystem gemäß einem der Ansprüche 1 bis 6, **dadurch gekennzeichnet, dass** der Körper (4) als rohrförmiger Hohlkörper ausgebildet ist, mit einem ersten durch eine erste Folie (9) verschlossenen Ende (5) und mit einem zweiten durch eine zweite Folie (10) verschlossenen Ende (6), wobei mindestens eine der zwei Folien (9, 10) aus dem plastischen Material (11) besteht.

8. Lanzettensystem gemäß Anspruch 7, **dadurch gekennzeichnet, dass** die erste Folie (9) durch ein Betätigungsmittel (18) geöffnet werden kann, das zur Bewegung der Lanzettenspitze (2) zu der zweiten Folie (10) hin dient, wobei die zweite Folie (10) aus dem plastischen Material (11) besteht und durch die Lanzettenspitze (2) durchstoßbar ist.

9. Lanzettensystem enthaltend mindestens zwei Lanzetten, die jeweils eine Lanzettenspitze und einen Lanzettenkörper aufweisen und jeweils in einzelnen, voneinander unabhängigen Kammern des Lanzettensystems enthalten sind, wobei jede Kammer zumindest eine Kammeröffnung aufweist, wobei die Kammeröffnung durch ein plastisches Material verschlossen ist, das von der Lanzettenspitze bei einem Stechvorgang zur Herstellung einer Austrittsöffnung durchstoßbar ist, **dadurch gekennzeichnet, dass** der Lanzettenkörper eine Verdickung aufweist, die so ausgebildet ist, dass sie die Austrittsöffnung bei dem Stechvorgang aufweitet, und dass die Lanzettenspitze nach dem Stechvorgang in die Kammer zurückbewegbar ist.

10. Stechhilfe enthaltend mindestens ein Lanzettensystem (1) gemäß einem der Ansprüche 1 bis 9 und ein Betätigungsmittel (18), das auf das Lanzettensystem (1) zur Bewegung der Lanzettenspitze (2) einwirken kann, so dass die Lanzettenspitze (2) zur Durchführung eines Stechvorganges das plastische Material (11) durchstoßen und aus der Austrittsöffnung (20) austreten kann.

## Claims

1. Lancet system (1), containing a lancet tip (2), a lancet body (3) and a body (4) which surrounds at least the lancet tip (2), the lancet tip (2) being movable in the body (4), and the body (4) containing a plastic material (11) which can be pierced by the lancet tip (2) during a pricking operation in order to produce an outlet opening (20), **characterized in that** the lancet body (3) has a thickened portion (14) which is designed in such a manner that it widens the outlet opening (20) during the pricking operation, and **in that** the lancet tip (2) can be moved back into the body (4) after the pricking operation.

2. Lancet system according to Claim 1, **characterized in that** the thickened portion (14) is a conical region (15) of the lancet body (3), the radius of which region increases with increasing distance from the lancet tip (2).

3. Lancet system according to either of Claims 1 and 2, **characterized in that** the thickened portion (14) is arranged in a region adjacent to the lancet tip (2).

4. Lancet system according to one of Claims 1 to 3, **characterized in that** the plastic material (11) contains at least one material selected from the group consisting of polyethylene, polypropylene, aluminum, tin and polytetrafluoroethylene.

5. Lancet system according to one of Claims 1 to 4, **characterized in that** the lancet system (1) contains a lancet needle (12) which comprises the lancet tip (2) and a needle body (13), the lancet body (3) at least partially surrounding the needle body (13).

6. Lancet system according to one of Claims 1 to 5, **characterized in that** the body (4) completely surrounds the lancet body (3) and the lancet tip (2), the lancet body (3) and the lancet tip (2) being displaceable together in the body (4).

7. Lancet system according to one of Claims 1 to 6, **characterized in that** the body (4) is designed as a tubular hollow body, with a first end (5) which is closed by a first film (9) and with a second end (6) which is closed by a second film (10), with at least one of the two films (9, 10) being composed of the plastic material (11).

8. Lancet system according to Claim 7, **characterized in that** the first film (9) can be opened by an actuating means (18) which serves to move the lancet tip (2) toward the second film (10), the second film (10) being composed of the plastic material (11) and being pierceable by the lancet tip (2).

9. Lancet system, containing at least two lancets which each have a lancet tip and a lancet body and are each contained in individual chambers, which are independent of each other, of the lancet system, each chamber having at least one chamber opening, and the chamber opening being closed by a plastic material which can be pierced by the lancet tip during a pricking operation in order to produce an outlet opening, **characterized in that** the lancet body has a thickened portion which is designed in such a manner that it widens the outlet opening during the pricking operation, and **in that** the lancet tip can be moved back into the chamber after the pricking operation.

10. Pricking aid, containing at least one lancet system (1) according to one of Claims 1 to 9 and an actuating means (18) which can act on the lancet system (1) in order to move the lancet tip (2) such that the lancet tip (2) can pierce the plastic material (11) and can emerge from the outlet opening (20) in order to carry out a pricking operation.

## Revendications

1. Système de lancette (1) contenant une pointe (2) de lancette, un corps (3) de lancette et un corps (4) qui entoure au moins la pointe (2) de lancette,
la pointe (2) de lancette pouvant se déplacer dans le corps (4),
le corps (4) contenant une matière plastique (11) qui peut être perforée par la pointe de lancette (2) lors d'une opération de perforation en vue d'établir une ouverture de sortie (20),
**caractérisé en ce que**
le corps de lancette (3) présente un épaississement (14) configuré de telle sorte qu'il agrandit l'ouverture de sortie (20) lors de l'opération de perforation et
**en ce que** la pointe (2) de lancette peut être rétractée dans le corps (4) après l'opération de perforation.

2. Système de lancette selon la revendication 1, **caractérisé en ce que** l'épaississement (14) est une partie conique (15) du corps (3) de lancette dont le rayon augmente lorsque la distance par rapport à la pointe (2) de lancette augmente.

3. Système de lancette selon l'une des revendications 1 ou 2, **caractérisé en ce que** l'épaississement (14) est disposé dans une partie adjacente à la pointe (2) de lancette.

4. Système de lancette selon l'une des revendications 1 à 3, **caractérisé en ce que** la matière plastique (11) contient au moins un matériau sélectionné dans l'ensemble constitué du polyéthylène, du polypropylène, de l'aluminium, de l'étain et du polytétrafluoroéthylène.

5. Système de lancette selon l'une des revendications 1 à 4, **caractérisé en ce que** le système de lancette (1) contient une aiguille (12) de lancette qui comporte la pointe de lancette (2) et un corps d'aiguille (13), le corps (3) de lancette entourant au moins une partie du corps d'aiguille (13).

6. Système de lancette selon l'une des revendications 1 à 5, **caractérisé en ce que** le corps (4) entoure complètement le corps de lancette (3) et la pointe de lancette (2), le corps de lancette (3) et la pointe de lancette (2) pouvant coulisser conjointement dans le corps (4).

7. Système de lancette selon l'une des revendications 1 à 6, **caractérisé en ce que** le corps (4) est configuré comme corps tubulaire creux qui présente une première extrémité (5) fermée par une première feuille (9) et une deuxième extrémité (6) fermée par une deuxième feuille (10), au moins l'une des deux feuilles (9, 10) étant constituée de la matière plastique (11).

8. Système de lancette selon la revendication 7, **caractérisé en ce que** la première feuille (9) peut être ouverte par un moyen d'actionnement (18) qui sert à déplacer la pointe de lancette (2) en direction de la deuxième feuille (10), la deuxième feuille (10) étant constituée de la matière plastique (11) et pouvant être perforée par la pointe de lancette (2).

9. Système de lancette contenant au moins deux lancettes qui présentent chacune une pointe de lancette et un corps de lancette et reprises chacune dans des chambres distinctes et séparées l'une de l'autre du système de lancette,
chaque chambre présentant au moins une ouverture de chambre,
l'ouverture de chambre étant fermée par une matière plastique qui peut être perforée par la pointe de lancette lors d'une opération de perforation en vue d'établir une ouverture de sortie,
**caractérisé en ce que**
le corps de lancette présente un épaississement configuré de manière à agrandir l'ouverture de sortie lors de l'opération de perforation et
**en ce que** la pointe de lancette peut être rétractée dans la chambre après l'opération de perforation.

10. Accessoire de perforation contenant au moins un système de lancette (1) selon l'une des revendications 1 à 9 et un moyen d'actionnement (18) qui peut agir sur le système de lancette (1) pour déplacer la pointe de lancette (2) de telle sorte que la pointe de lancette (2) puisse perforer la matière plastique (11) en vue d'exécuter une opération de perforation et puisse sortir hors de l'ouverture de sortie (20).
